# EUROPÄISCHE PATENTANMELDUNG

(11) **EP 4 707 771 A1**
(43) Veröffentlichungstag der Anmeldung: **11.03.2026**
(21) Anmeldenummer: 25199699.7
(22) Anmeldetag: 02.09.2025
(51) Int. Cl.: G01N 1/22, G01N 33/00

(54) **GASPROBENSAMMELVORRICHTUNG**

(30) Priorität: 04.09.2024 DE 102024002850
(71) Anmelder: Universität Basel, 4001 Basel (CH); EAWAG, Eidgenössische Anstalt für Wasserversorgung, Abwasserreinigung und Gewässerschutz, 8600 Dübendorf (CH)
(72) Erfinder: Schilling, Oliver Stephan, 8932 Mettmenstetten (CH); Tomonaga, Yama, 8610 Uster (CH)
(74) Vertreter: IPrime Rentsch Kaelin AG

(57) **Zusammenfassung**

Die vorliegende Erfindung ist auf eine Gasprobensammelvorrichtung (1) mit einem Gas-Gleichgewichts-Membraneinlass für die Quantifizierung von gelösten Gasen in lösungsmittelhaltigen Lösungen gerichtet. Zudem betrifft die Erfindung ein Verfahren für die Quantifizierung von gelösten Gasen in lösungsmittelhaltigen Lösungen mittels der erfindungsgemässen Vorrichtung sowie die Verwendung der Vorrichtung in diesem Verfahren.

## Beschreibung

Die vorliegende Erfindung ist auf eine Gasprobensammelvorrichtung (1) mit einem Gas-Gleichgewichts-Membraneinlass für die Quantifizierung von gelösten Gasen in lösungsmittelhaltigen Lösungen gerichtet. Zudem betrifft die Erfindung ein Verfahren für die Quantifizierung von gelösten Gasen in lösungsmittelhaltigen Lösungen mittels der erfindungsgemässen Vorrichtung sowie die Verwendung der Vorrichtung in diesem Verfahren.

In wässrigen und nicht-wässrigen Lösungen gelöste Gase werden routinemässig mittels Gasanalysegeräten wie beispielsweise Massenspektrometern, Gaschromatographen, Alpha-Spektrometern, «cavity-ring down»-Spektrometern, oder anderen optischen Analysegeräten, etc. quantifiziert. Einige dieser Gasanalysevorrichtungen nutzen Kapillargaseinlässe und verwenden eine gasdurchlässige Membran zur Abtrennung der Lösungsmittelflüssigkeit vom Gas für den Einlass in die Analysevorrichtung. Ein Beispiel ist die sogenannte Membraneinlassmassenspektrometrie (membrane-inlet mass spectrometry, MIMS). Die Gas-Gleichgewichts (GE)-MIMS (GE-MIMS) ist eine spezielle MIMS-Technik für die einfache quantitative Analyse von Teildrücken von in Flüssigkeit gelösten Gasspezies (Brennwald et al., Environ. Sci. Technol. 2016, 50, 24, 13455-13463; Mächler et al., Environ. Sci. Technol. 2012, 46, 15, 8288-8296; Cassar et al., Anal. Chem. 2009, 81 (5), 1855-1864; Manning et al., Anal. Chem. 2016, 88 (6), 3040-3048). Typisch für die GE-MIMS-Technik ist, dass ein Löslichkeitsgleichgewicht zwischen der Probenflüssigkeit und dem Gasüberstand mittels einer Membran dazwischen eingestellt wird. Ein kleiner Anteil der Gase oberhalb der Membran im Gasüberstand fliesst durch eine Kapillare oder eine andere druckreduzierende Vorrichtung in das Vakuumsystem einer Gasanalysenvorrichtung, wo diese Gase quantitativ analysiert werden.

Um die Werte der Gasanalysenvorrichtung in Bezug auf die Teildrücke des gelösten Gases genau zu kalibrieren, muss die Geschwindigkeit des Gasflusses vom Gasüberstand über die Kapillare in die Gasanalysenvorrichtung bekannt sein, die jedoch eine komplizierte Funktion ist, die stark und in nicht-linearer Weise vom Gesamtgasdruck am Kapillareingang, der Viskosität des Gases (die wiederum von der Temperatur und der Zusammensetzung des Gases abhängt) und weiteren Parametern abhängt (siehe G.M. Fryer: A theory of gas flow through capillary tubes. Proc. R. Soc. London, Ser. A, 1966, 293, 329-341).

In den meisten praktischen Anwendungen sind die genannten Parameter variabel und a-priori unbekannt. Daher ist die Quantifizierung des Gasflusses durch eine Kapillare notorisch schwierig, was wiederum die akkurate Kalibrierung der Gasanalysenergebnisse in Bezug auf die Teildrücke des gelösten Gases herausfordernd gestaltet.

Als ein Ergebnis haben die derzeitigen Gasgleichgewichts-Membraneinlass-Gasanalysengeräte (GE-MIMS), und damit einhergehende Analyseverfahren das inhärente Problem, dass jegliche Änderungen in den Parametern der zu analysierenden Flüssigprobe wie beispielsweise Temperatur- und Zusammensetzungsänderungen eine oder mehrere Kalibrierungen der Vorrichtung erfordern, insbesondere wenn eine kontinuierliche Probennahme mit sich ändernden Parametern vorgesehen ist.

EP4109092 B1 offenbart ein Verfahren zur quantitativen Bestimmung von in Flüssigkeit gelösten Gasen nach Abtrennung der Gasphase über der Flüssigkeit mittels gaspermeabler Membran im Gleichgewichtszustand aus dem Gesamtdruck aller freien Gase, den Teildrücken der abgetrennten Gase mittels Gasanalysenvorrichtung, und der Lösungstemperatur, wobei die Zuführung des Gases von der Membran zu der Analysevorrichtung üblicherweise druckreduzierend, z.B. mittels Kapillaren ausgeführt ist. Problematisch ist hierbei, dass das Analysengerät, meist ein Massenspektrometer, und das Probennahmegerät mit gaspermeabler Membran mittels Kapillarzuführung druckreduzierend und dauerhaft klobig miteinander verbunden sind und die Auswertung letztendlich unabhängig vom Gleichgewichtsdruck in der Probenvorrichtung erfolgt. DE102013112823B3 offenbart eine Vorrichtung und ein Verfahren zur Detektion von Gas in mit Isoliermedium gefüllten Hochspannungsgeräten, insbesondere in Hochspannungstransformatoren, wobei das auf Gasanteil zu messende Isoliermedium statisch in dem Hochspannungsgerät vorliegt und ein Trägergas in einer gasdurchlässigen Kapillare in dem Isoliermedium das zu messende Gas aufnimmt und zur Messkammer befördert.

Es ist die Aufgabe der vorliegenden Erfindung, eine Gasprobensammelvorrichtung mit einem Gas-Flüssigkeit-Gleichgewichts-Membraneinlass für die spätere Quantifizierung von gelösten Gasen in lösungsmittelhaltigen Lösungen, optional wässrigen Lösungen, zur Verfügung zu stellen, die gut handhabbar ist und die Parameter des Gases in der flüssigen Probe bzw. im Gas-Flüssigkeits-Gleichgewichtszustand zum Zeitpunkt der Probennahme festlegt.

Diese Aufgabe wird mittels einer Gasprobensammelvorrichtung (1) mit einem Gas-Gleichgewichts-Membraneinlass für die Quantifizierung von gelösten Gasen in lösungsmittelhaltigen Lösungen, optional wässrigen Lösungen, bei Gleichgewichtsbedingungen gelöst, wobei die Vorrichtung Folgendes umfasst:
(a) einen gasundurchlässigen Probensammelbehälter (2) mit einer gasdurchlässigen Membran (3) zur Abtrennung gelöster Gase aus einer lösungsmittelhaltigen Lösung, optional einer wässrigen Lösung (L), wobei die Membran (3) das Behältervolumen in ein konstantes Gasvolumen (4) und ein konstantes Flüssigkeitsvolumen (5) trennt, wobei das Flüssigkeitsvolumen (5) dazu ausgelegt ist, von einer lösungsmittelhaltigen Lösung (L), optional einer wässrigen Lösung, bei Gas-Flüssigkeits-Gleichgewichtsbedingungen durchströmt zu werden,
(b) optional einen Drucksensor (6), der zur Bestimmung des Gesamtgasdrucks *p*ₜₒₜ der abgetrennten Gase in dem Gasvolumen (4) bei den Gleichgewichtsbedingungen ausgelegt ist,
(c) optional einen Temperatursensor (7), der zur Bestimmung der Temperatur der lösungsmittelhaltigen Lösung in dem Flüssigkeitsvolumen bei Gleichgewichtsbedingungen ausgelegt ist,
(d) einen gasdichten Gassammelbehälter (8) mit einem gasdicht verschliessbaren Eingang (9), und einem gasdicht verschliessbaren Ausgang (10),
(e) eine Zuleitung (11) für das Zuführen wenigstens eines Teils von allen abgetrennten Gasen zum Eingang des Gassammelbehälters (9),
(f) eine Ableitung (12) für das Abführen wenigstens eines Teils von allen abgetrennten Gasen zurück zum Gasvolumen (4) des Probensammelbehälters (2),
(g) eine Zirkulationsgaspumpe (13), optional vor, im oder dem Gassammelbehälter (8) nachgeordnet, die das Gas aus dem Gassammelbehälter (8) zu dem Gasvolumen (4) des Probensammelbehälters (2) zurückführt,
wobei die Vorrichtung so ausgelegt ist, dass im Betrieb der Gesamtgasdruck *p*ₜₒₜ der abgetrennten Gase bei den Gleichgewichtsbedingungen in dem Gassammelbehälter (8), in dem Probensammelbehälter (2), in der Zuleitung (11) und in der Ableitung (12) konstant bleibt, und wobei der gasundurchlässige Probensammelbehälters (2) einen Einlass und einen Auslass aufweist, wobei die Flüssigkeit L in den Eingang einströmt, die gasdurch-lässige Membran (3) in dem Probensammelbehälter (2) entlang strömt, und am Auslass aus dem Probensammelbehälters (2) ausströmt.

Die Gasprobensammelvorrichtung der Erfindung verfügt über einen gasundurchlässigen Probensammelbehälter (2) mit einer gaspermeablen Membran, also einen Gas-Gleichgewichts-Membraneinlass, der die flüssige Probe aufnimmt und Gase über die gasdurchlässige Membran freisetzt, bis ein Gleichgewicht von in der Flüssigkeit gelösten Gasen auf der einen Seite und freien Gasen auf der anderen Seite der Membran entsteht. Der Gleichgewichtszustand ist im Wesentlichen abhängig von der Temperatur, dem Lösungsmittel, dem Druck und der Zusammensetzung der Gase. Dieser Gasprobensammelbehälter ist so ausgestaltet, dass die Lösung (L), optional eine wässrige Lösung, den Behälter bis zur Entstehung der Gas-Flüssigkeits-Gleichgewichtsbedingungen durchströmt und die Gasmenge in der Lösung und die freie Gasmenge in dem Behälter konstant sind.

Die Gasprobensammelvorrichtung kann optional einen Drucksensor (6) zur Bestimmung des Gesamtgasdrucks *p*ₜₒₜ der abgetrennten Gase in dem Gasvolumen (4) bei den Gleichgewichtsbedingungen der Probennahme aufweisen. Allerdings kann der Gesamtdruck der Gase alternativ noch später aus der entnommenen Gasprobe vor Einlass in die Messvorrichtung bestimmt werden.

Die Gasprobensammelvorrichtung kann optional auch einen Temperatursensor (7) umfassen, der zur Bestimmung der Temperatur der lösungsmittelhaltigen Lösung in dem Flüssigkeitsvolumen bei Gleichgewichtsbedingungen ausgelegt ist. Alternativ dazu kann diese Temperatur aber auch in der Umgebung der Probennahme bestimmt werden, z.B. mit einem konventionellen Thermometer.

Die Gasprobensammelvorrichtung umfasst des Weiteren neben dem Probensammelbehälter einen gasdichten Gassammelbehälter (8) mit einem gasdicht verschliessbaren Eingang (9), und einem gasdicht verschliessbaren Ausgang (10), wobei der Probensammelbehälter über eine Zuleitung (11) für das Zuführen wenigstens eines Teils von allen abgetrennten Gasen zum Eingang des Gassammelbehälters (9) verfügt, sowie der Gassammelbehälter über eine Ableitung (12) für das Abführen wenigstens eines Teils von allen abgetrennten Gasen zurück zum Gasvolumen (4) des Probensammelbehälters (2) verfügt. Es entsteht so eine Zu- und Rückführung der freien Gase zwischen Gasprobensammelvorrichtung mit Membran und dem Gassammelbehälter bis zur Einstellung des Gleichgewichts zwischen flüssiger und Gasphase auf beiden Seiten der gasdurchlässigen Membran. Die Zu- und Ableitungen können beliebig ausgeführt werden, beispielsweise als Röhren oder Schläuche, beispielsweise in starrem Metall oder aus Kunststoff oder flexibel sein. Es ist lediglich darauf zu achten, dass die Leitungen ausreichend gasdicht sind, um ein Entweichen des Gases in dem Umfang zu vermeiden, dass das Gas-Flüssigkeits-Gleichgewicht beeinträchtigt wird.

Die Gasprobensammelvorrichtung der Erfindung umfasst ferner eine Zirkulationsgaspumpe (13), die das Gas aus dem Gassammelbehälter (8) zu dem Gasvolumen (4) des Probensammelbehälters (2) zurückführt. Die Zirkulationspumpe (13) kann optional im Gassammelbehälter (8), davor oder danach angeordnet sein, beispielsweise am Anfang, innerhalb oder am Ende der Zu-(11), am Anfang, innerhalb oder am Ende der Ableitung (12) angeordnet sein. Vorzugsweise befindet sich die Zirkulationspumpe nicht im gasundurchlässigen Probensammelbehälter (2). Die Zirkulationsgaspumpe kann jegliche Geometrie oder Ausführungsform aufweisen, die zur Beschleunigung des Gasstroms über die Zu- und Ableitungen geeignet ist. Die Beschleunigung des Gasstroms sollte so eingestellt sein, dass die Gleichgewichtsbedingungen im Probensammelbehälter sich schneller als bei lediglich Diffusion einstellen, nicht aber durch den entstehenden Gasdruck beeinträchtigt werden.

Die Vorrichtung ist für den Probensammelbetrieb so zu dimensionieren und auszulegen, dass im Betrieb der Gesamtgasdruck *p*ₜₒₜ der abgetrennten Gase bei den Gleichgewichtsbedingungen in dem Gassammelbehälter (8), in dem Probensammelbehälter (2), in der Zuleitung (11) und in der Ableitung (12) konstant bleibt.

Wenn der Gleichgewichtszustand erreicht ist, meist innerhalb weniger Minuten, kann die Probe in dem Gassammelbehälter (8) mittels Ventilen der Zu- und Ableitungen gasdicht verschlossen werden und der Gassammelbehälter (8) von den Zu- und Ableitungen getrennt werden und zu einer Analysenvorrichtung für Gase transportiert und daran angeschlossen werden. Für eine mehrfache Probennahme können mehrere Gassammelbehälter nacheinander an die Zu- und Ableitungen eines Gasprobenbehälters angeschlossen werden. Die Temperatur der Probe und der Gasgesamtdruck in dem Probensammelbehälter können mittels Sensoren in oder an der Vorrichtung bestimmt werden oder von der Vorrichtung getrennt bestimmt werden. Zum Beispiel kann der Gasgesamtdruck mittels des Gases in dem Gassammelbehälter bestimmt werden, wenn die Temperatur während der Probennahme gemessen und aufgezeichnet wurde.

Die Berechnung der Mengen und Anteile der Gase in den mittels der Vorrichtung der Erfindung genommen Gasproben kann beispielsweise entsprechend den in der EP4109092 B1 offenbarten Berechnungsverfahren bestimmt werden. Die erfindungsgemässe Vorrichtung und die in der EP4109092 B1 offenbarten Berechnungsverfahren sind letztendlich für die Quantifizierung beliebiger Gasproben geeignet, zum Beispiel für die Quantifizierung von gelösten Gasen sowie deren Isotopenzusammensetzung, ausgewählt aus der Gruppe, bestehend aus He, Ne, Ar, Kr, Xe, Rn, N₂, O₂, CO₂, CH₄, C₂H₆, C₃H₈, SF₆ und H₂.

Ein weiterer Aspekt der vorliegenden Erfindung ist auf ein Verfahren für die Quantifizierung von gelösten Gasen in lösungsmittelhaltigen Lösungen, optional wässrigen Lösungen, gerichtet, das Folgendes umfasst:
(i) das Abtrennen und Sammeln gelöster Gase aus einer lösungsmittelhaltigen Lösung mittels einer Gasprobensammelvorrichtung (1) der vorliegenden Erfindung bei Gas-Flüssigkeits-Gleichgewichtsbedingungen,
(ii) das Bestimmen des Gesamtgasdrucks *p*ₜₒₜ der abgetrennten Gase bei Gleichgewichtsbedingungen im Probensammelbehälter (2) oder im gasdichten Gassammelbehälter (8),
(iii) das Bestimmen des Lösungsmittelpartialdrucks *p*ₛₒₗᵥ in den abgetrennten Gasen aus dem Gassammelbehälter (8), optional aus der Lösungsmitteltemperatur bei den Gleichgewichtsbedingungen,
(iv) die Zuführung wenigstens eines Teils von allen abgetrennten Gasen aus dem Gassammelbehälter (8) zu einer Gasanalysenvorrichtung, optional durch druckreduzierende Mittel, optional durch Kapillarmittel.

Optional kann die Zuführung eines Teils der abgetrennten Gase aus dem Gassammelbehälter (8) wiederholt erfolgen, zum Beispiel, um sukzessive Messungen an verschiedenen Gasanalysenvorrichtungen auszuführen.

Für das Verfahren der Erfindung kann die zu verwendende Gasanalysenvorrichtung einen kapillar-basierten Gaseinlass aufweisen, und optional aus der Gruppe ausgewählt sein, die aus einem Massenspektrometer, Alpha-Spektrometern, optischen Spektrometern, «cavity-ring down»-Spektrometern, und einem Gaschromatographen besteht.

Das Verfahren der Erfindung ist auf im Wesentlichen alle in Lösungsmittel gelösten bzw. lösbaren Gase anwendbar, insbesondere zur Quantifizierung von gelösten Gasen sowie deren Isotopenzusammensetzung, wie beispielsweise von He, Ne, Ar, Kr, Xe, Rn, N₂, O₂, CO₂, CH₄, C₂H₆, C₃H₈, SF₆ und H₂.

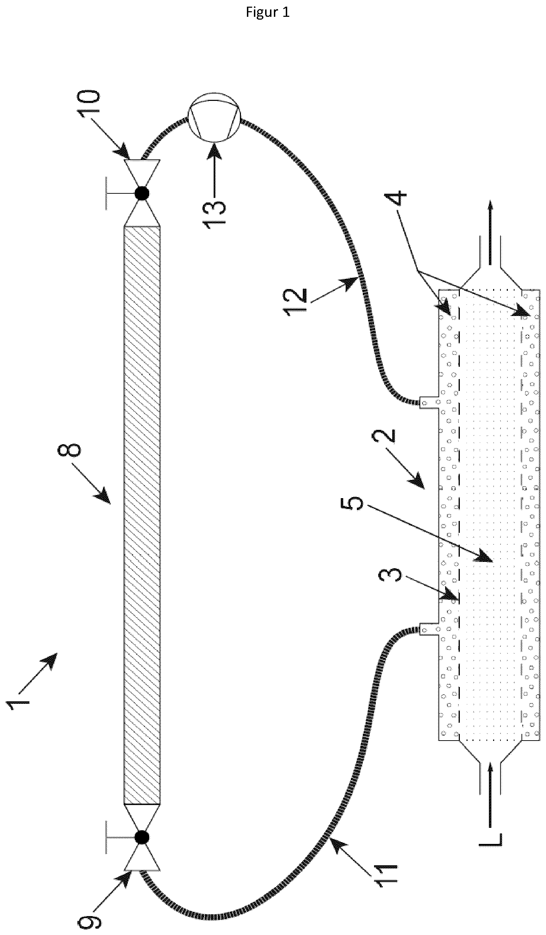

Ein weiterer Aspekt der Erfindung bezieht sich auf die Verwendung einer Vorrichtung der Erfindung oder eines Verfahrens der Erfindung zur Quantifizierung von Teildrücken von gelösten Gasen in lösungsmittelhaltigen Lösungen, optional in wässrigen Lösungen. Beispielsweise betrifft diese Verwendung lösungsmittelhaltige Lösungen und optional wässrige Lösungen wie Trinkwasser, Abwasser, Schmutzwasser, Naturwasser, Quellwasser, Meerwasser, Seewasser, biologischen Flüssigkeiten, Urin, Blut, oder nicht-wässrigen Lösungen, optional ausgewählt aus alkoholischen Lösungen, mineralischen und biologischen Ölen, Benzin, Bremsflüssigkeit und Hydraulikflüssigkeiten. Die Vorrichtung der Erfindung wird in der Figur 1 beispielhaft und nicht die Ansprüche beschränkend erläutert.

### Bezugszeichenliste

- 1: Gasprobensammelvorrichtung
- 2: gasundurchlässiger Probensammelbehälter
- 3: gasdurchlässige Membran
- 4: konstantes Gasvolumen
- 5: konstantes Flüssigkeitsvolumen
- 6: optionaler Drucksensor (nicht gezeigt)
- 7: optionaler Temperatursensor (nicht gezeigt)
- 8: gasdichter Gassammelbehälter
- 9: gasdichter verschliessbarer Eingang des Gassammelbehälters
- 10: gasdicht verschliessbarer Ausgang des Gassammelbehälters
- 11: Zuleitung
- 12: Ableitung
- 13: Zirkulationsgaspumpe
- L: gashaltiges Lösungsmittel

## Patentansprüche

1. Eine Gasprobensammelvorrichtung (1) mit einem Gas-Gleichgewichts-Membraneinlass für die Quantifizierung von gelösten Gasen in lösungsmittelhaltigen Lösungen, optional wässrigen Lösungen, bei Gleichgewichtsbedingungen, wobei die Vorrichtung Folgendes umfasst:
(a) einen gasundurchlässigen Probensammelbehälter (2) mit einer gasdurchlässigen Membran (3) zur Abtrennung gelöster Gase aus einer lösungsmittelhaltigen Lösung, optional einer wässrigen Lösung, wobei die Membran (3) das Behältervolumen in ein konstantes Gasvolumen (4) und ein konstantes Flüssigkeitsvolumen (5) trennt, wobei das Flüssigkeitsvolumen (5) dazu ausgelegt ist, von einer lösungsmittelhaltigen Lösung (L), optional einer wässrigen Lösung, bei Gas-Flüssigkeits-Gleichgewichtsbedingungen durchströmt zu werden,
(b) optional einen Drucksensor (6), der zur Bestimmung des Gesamtgasdrucks *p*ₜₒₜ der abgetrennten Gase in dem Gasvolumen (4) bei den Gleichgewichtsbedingungen ausgelegt ist,
(c) optional einen Temperatursensor (7), der zur Bestimmung der Temperatur der lösungsmittelhaltigen Lösung in dem Flüssigkeitsvolumen bei Gleichgewichtsbedingungen ausgelegt ist,
(d) einen gasdichten Gassammelbehälter (8) mit einem gasdicht verschliessbaren Eingang (9), und einem gasdicht verschliessbaren Ausgang (10),
(e) eine Zuleitung (11) für das Zuführen wenigstens eines Teils von allen abgetrennten Gasen zum Eingang des Gassammelbehälters (9),
(f) eine Ableitung (12) für das Abführen wenigstens eines Teils von allen abgetrennten Gasen zurück zum Gasvolumen (4) des Probensammelbehälters (2),
(g) eine Zirkulationsgaspumpe (13), die das Gas aus dem Gassammelbehälter (8) zu dem Gasvolumen (4) des Probensammelbehälters (2) zurückführt,
wobei die Vorrichtung so ausgelegt ist, dass im Betrieb der Gesamtgasdruck *p*ₜₒₜ der abgetrennten Gase bei den Gleichgewichtsbedingungen in dem Gassammelbehälter (8), in dem Probensammelbehälter (2), in der Zuleitung (11) und in der Ableitung (12) konstant bleibt, und
wobei der gasundurchlässige Probensammelbehälters (2) einen Einlass und einen Auslass aufweist, wobei die Flüssigkeit L in den Eingang einströmt, die gasdurch-lässige Membran (3) in dem Probensammelbehälter (2) entlang strömt, und am Auslass aus dem Probensammelbehälters (2) ausströmt.

2. Die Vorrichtung von Anspruch 1, wobei die Vorrichtung für die Quantifizierung von gelösten Gasen sowie deren Isotopenzusammensetzung, ausgewählt aus der Gruppe, bestehend aus He, Ne, Ar, Kr, Xe, Rn, N₂, O₂, CO₂, CH₄, C₂H₆, C₃H₈, SF₆ und H₂, ausgelegt ist.

3. Ein Verfahren für die Quantifizierung von gelösten Gasen in lösungsmittelhaltigen Lösungen, optional wässrigen Lösungen, das Folgendes umfasst:
(i) das Abtrennen und Sammeln gelöster Gase aus einer lösungsmittelhaltigen Lösung mittels einer Gasprobensammelvorrichtung (1) nach einem der Ansprüche 1 oder 2 bei Gas-Flüssigkeits-Gleichgewichtsbedingungen,
(ii) das Bestimmen des Gesamtgasdrucks *p*ₜₒₜ der abgetrennten Gase bei Gleichgewichtsbedingungen im Probensammelbehälter (2) oder im gasdichten Gassammelbehälter (8),
(iii) das Bestimmen des Lösungsmittelpartialdrucks *p*ₛₒₗᵥ in den abgetrennten Gasen aus dem Gassammelbehälter (8), optional aus der Lösungsmitteltemperatur bei den Gleichgewichtsbedingungen,
(iv) die Zuführung wenigstens eines Teils von allen abgetrennten Gasen aus dem Gassammelbehälter (8) zu einer Gasanalysenvorrichtung, optional durch druckreduzierende Mittel, optional durch Kapillarmittel.

4. Das Verfahren von Anspruch 3, wobei die Gasanalysenvorrichtung einen kapillar-basierten Gaseinlass aufweist, und optional aus der Gruppe ausgewählt ist, die aus einem Massenspektrometer, Alpha-Spektrometern, optischen Spektrometern, «cavity-ring down»-Spektrometern, und einem Gaschromatographen besteht.

5. Das Verfahren von Anspruch 3 zur Quantifizierung von gelösten Gasen sowie deren Isotopenzusammensetzung, ausgewählt aus der Gruppe, bestehend aus He, Ne, Ar, Kr, Xe, Rn, N₂, O₂, CO₂, CH₄, C₂H₆, C₃H₈, SF₆ und H₂.

6. Verwendung einer Vorrichtung gemäss einem der Ansprüche 1 oder 2 oder eines Verfahrens gemäss einem der Ansprüche 3 oder 4 für die Quantifizierung von Teildrücken von gelösten Gasen in lösungsmittelhaltigen Lösungen, optional in wässrigen Lösungen.

7. Die Verwendung von Anspruch 6 für die Quantifizierung von gelösten Gasen sowie deren Isotopenzusammensetzung, ausgewählt aus der Gruppe, bestehend aus He, Ne, Ar, Kr, Xe, Rn, N₂, O₂, CO₂, CH₄, C₂H₆, C₃H₈, SF₆ und H₂.

8. Die Verwendung gemäss einem der Ansprüche 6 oder 7, wobei die lösungsmittelhaltigen Lösungen wässrige Lösungen sind, optional ausgewählt aus der Gruppe bestehend aus Trinkwasser, Abwasser, Schmutzwasser, Naturwasser, Thermalwasser, Mineralwasser, Grundwasser, Fluss- und Bachwasser, Quellwasser, Regenwasser, Süsswasser, Meerwasser, Seewasser, biologischen Flüssigkeiten, Urin, Blut, oder nicht-wässrigen Lösungen, optional ausgewählt aus alkoholischen Lösungen, mineralischen und biologischen Ölen, Benzin, Bremsflüssigkeit und Hydraulikflüssigkeiten.
